# EUROPEAN PATENT APPLICATION

(11) **EP 4 670 639 A1**
(43) Date of publication of application: **31.12.2025**
(21) Application number: 24760370.7
(22) Date of filing: 20.02.2024
(51) Int. Cl.: A61B 6/08, A61B 6/00

(54) **INFORMATION PROCESSING DEVICE, INFORMATION PROCESSING METHOD, AND INFORMATION PROCESSING PROGRAM**

(30) Priority: 21.02.2023 JP 2023025466
(71) Applicant: FUJIFILM Corporation, Tokyo 106-8620 (JP)
(72) Inventor: SUGAHARA, Masataka, Tokyo 106-8620 (JP)
(74) Representative: Dehns Germany Partnerschaft mbB
(86) International application number: PCT/JP2024/006071
(87) International publication number: WO 2024/177072

(57) **Abstract**

An information processing apparatus including at least one processor, in which the processor is configured to acquire at least one optical image obtained by optical imaging of a subject, extract a feature point of the subject based on the optical image, specify a target imaging region that is a target in a case in which the subject undergoes radiography from a direction substantially the same as an imaging direction of the optical imaging in the optical image, based on the feature point, generate a superimposed image in which the target imaging region is superimposed on the optical image, and perform control of displaying the superimposed image on a display.

## Description

### Technical Field

The present disclosure relates to an information processing apparatus, an information processing method, and an information processing program.

### Background Art

In the related art, in radiography, a technology of supporting positioning based on an optical image obtained by optical imaging of a subject is known. For example, JP2014-117368A discloses a technology of guiding reproduction of imaging under the same imaging conditions and the same positioning as in past imaging at a present point in time based on an optical image of a subject and imaging conditions at a past point in time.

### SUMMARY OF INVENTION

In recent years, in radiography, there has been an increasing demand for obtaining a high-quality radiation image in which an imaging part of a subject is appropriately imaged. For this purpose, registration of the radiation source, the radiation detector, and the subject is important. In addition, it is required to perform positioning for the posture of the subject as predetermined in the guideline or the like.

The present disclosure provides an information processing apparatus, an information processing method, and an information processing program capable of supporting high-quality radiography.

A first aspect of the present disclosure is an information processing apparatus comprising at least one processor, in which the processor is configured to acquire at least one optical image obtained by optical imaging of a subject, extract a feature point of the subject based on the optical image, specify a target imaging region that is a target in a case in which the subject undergoes radiography from a direction substantially the same as an imaging direction of the optical imaging in the optical image, based on the feature point, generate a superimposed image in which the target imaging region is superimposed on the optical image, and perform control of displaying the superimposed image on a display.

In the first aspect, the processor may be configured to extract a plurality of feature points of the subject based on the optical image, and specify the target imaging region based on a relative positional relationship among the plurality of feature points.

In the first aspect, the processor may be configured to specify at least one predetermined reference feature point among the plurality of feature points, and specify the target imaging region based on the reference feature point.

In the first aspect, the processor may be configured to acquire imaging part information indicating an imaging part of the radiography, and specify the reference feature point corresponding to the imaging part information.

In the first aspect, the processor may be configured to generate the superimposed image using a trained model that is trained in advance to receive at least one of the optical image or the feature point as an input and to output the target imaging region or the superimposed image.

In the first aspect, the processor may be configured to issue a warning in a case in which a feature point other than a predetermined feature point among the plurality of feature points is included in the target imaging region or is located within a predetermined range from the target imaging region.

In the first aspect, the processor may be configured to determine whether the subject takes predetermined positioning based on a positional relationship among the plurality of feature points, and issue a warning in a case in which it is determined that the subject does not take predetermined positioning.

In the first aspect, the processor may be configured to acquire at least one determination optical image obtained by optical imaging of the subject from a direction different from the imaging direction of the optical image, extract a plurality of determination feature points of the subject based on the determination optical image, determine whether the subject takes predetermined positioning based on a positional relationship among the plurality of determination feature points, and issue a warning in a case in which it is determined that the subject does not take predetermined positioning.

In the first aspect, the optical image may be at least one of a visible light image or a distance image representing a distance to the subject.

A second aspect of the present disclosure is an information processing method including acquiring at least one optical image obtained by optical imaging of a subject, extracting a feature point of the subject based on the optical image, specifying a target imaging region that is a target in a case in which the subject undergoes radiography from a direction substantially the same as an imaging direction of the optical imaging in the optical image, based on the feature point, generating a superimposed image in which the target imaging region is superimposed on the optical image, and performing control of displaying the superimposed image on a display.

A third aspect of the present disclosure is an information processing program that causes a computer to execute a process including acquiring at least one optical image obtained by optical imaging of a subject, extracting a feature point of the subject based on the optical image, specifying a target imaging region that is a target in a case in which the subject undergoes radiography from a direction substantially the same as an imaging direction of the optical imaging in the optical image, based on the feature point, generating a superimposed image in which the target imaging region is superimposed on the optical image, and performing control of displaying the superimposed image on a display.

According to the above-described aspects, the information processing apparatus, the information processing method, and the information processing program of the present disclosure can support high-quality radiography.

### BRIEF DESCRIPTION OF DRAWINGS

Fig. 1 is a diagram showing an example of a schematic configuration of an imaging system.
Fig. 2 is a schematic view showing an example of a usage aspect of the imaging apparatus.
Fig. 3 is a block diagram showing an example of a hardware configuration of a console.
Fig. 4 is a block diagram showing an example of a functional configuration of the console.
Fig. 5 is a view showing an example of an optical image.
Fig. 6 is a diagram showing an example of a feature point.
Fig. 7 is a diagram showing an example of a target imaging region.
Fig. 8 is a diagram showing an example of a determination optical image.
Fig. 9 is an example of the screen displayed on the display.
Fig. 10 is a flowchart showing an example of information processing.

### DESCRIPTION OF EMBODIMENTS

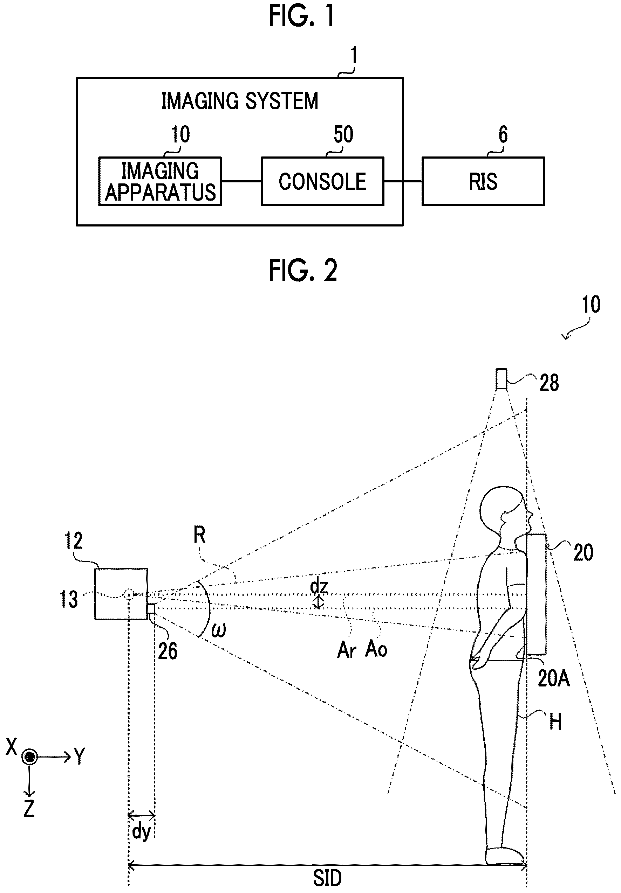

Hereinafter, embodiments of the present disclosure will be described with reference to drawings. First, a configuration of an imaging system 1 will be explained with reference to Fig. 1. Fig. 1 is a view showing an example of a schematic configuration of the imaging system 1. As shown in Fig. 1, the imaging system 1 comprises an imaging apparatus 10 and a console 50. The imaging apparatus 10 and the console 50, and the console 50 and an external radiology information system (RIS) 6 are configured to be connectable to each other via a wired or wireless network.

The console 50 acquires an imaging order or the like from the RIS 6 and controls the imaging apparatus 10 according to the acquired imaging order, an instruction from a user, and the like. The imaging apparatus 10 captures a radiation image of a subject H according to the control of the console 50. The console 50 is an example of an information processing apparatus of the present disclosure.

Next, the imaging apparatus 10 will be explained with reference to Fig. 2. Fig. 2 is a diagram showing a schematic configuration of the imaging apparatus 10. As shown in Fig. 2, the imaging apparatus 10 comprises a radiation emitting unit 12, a radiation detector 20, a first optical camera 26, and a second optical camera 28. Fig. 2 shows a state in which the chest of the subject H undergoes radiography as the imaging part, as an example.

The radiation emitting unit 12 comprises a radiation source 13 that emits radiation R such as X-rays. In addition, the radiation emitting unit 12 comprises a collimator (not shown) and the like, and is configured to change an irradiation field (the range illustrated by the two-dot chain line in Fig. 2) of the radiation R emitted from the radiation source 13. The type of the radiation source 13 is not particularly limited, and for example, a radiation source such as a hot cathode type or a cold cathode type can be appropriately applied.

For example, the radiation emitting unit 12 may be a so-called ceiling-running type emitting unit that is held by a support column suspended from a ceiling of the imaging room. In the ceiling-running type emitting unit, a support column that is expandable and contractible in the vertical direction (Z direction) is attached to rails running around the ceiling via wheels, and the emitting unit is movable in the horizontal direction (X direction and Y direction) in the imaging room. By the movement of the support column in the horizontal direction and the expansion and contraction of the support column in the vertical direction, the radiation emitting unit 12 is also translationally moved in the horizontal direction and the vertical direction. In addition, the radiation emitting unit 12 may be movable rotationally around a rotational axis extending in the horizontal direction, or may be movable rotationally around a rotational axis extending in the vertical direction.

In addition, for example, the radiation emitting unit 12 may be a portable emitting unit. The portable emitting unit may be used for, for example, a simple radiographic examination in a medical facility, a radiographic examination in a case of home medical care, a radiographic examination outdoors, an on-site medical care in a disaster-stricken area or a medically underserved area, or the like. In addition, for example, the radiation emitting unit 12 may be a stationary emitting unit installed in an imaging room.

The radiation detector 20 detects the radiation R transmitted through the subject H on a detection surface 20A, generates a radiation image based on the detected radiation R, and outputs image data indicating the generated radiation image. The radiation detector 20 may be, for example, a portable electronic cassette, and may be used by being placed on any base or held by the subject H. That is, the radiation detector 20 may be movable with respect to the radiation emitting unit 12 in a horizontal direction (X direction and Y direction) and a vertical direction (Z direction) to any position. In addition, for example, the radiation detector 20 may be a stationary type that is disposed inside an imaging table installed in the imaging room.

The type of the radiation detector 20 is not particularly limited. For example, the radiation detector 20 may be an indirect conversion type radiation detector that converts the radiation R into light and converts the converted light into electric charge, or may be a direct conversion type radiation detector that directly converts the radiation R into electric charge.

The first optical camera 26 and the second optical camera 28 are, for example, optical digital cameras that perform imaging based on visible light and are configured to include a complementary metal oxide semiconductor (CMOS) type image sensor, a charge coupled device (CCD) type image sensor, or the like. The first optical camera 26 and the second optical camera 28 can capture a still image and/or a video.

The first optical camera 26 images a region (the range illustrated by the one-dot chain line in Fig. 2) wider than the irradiation field of the radiation R (the range illustrated by the two-dot chain line in Fig. 2), and generates an optical image 30. In addition, an angle of view ω of the first optical camera 26 is stored in the storage unit 52 in advance. As shown in Fig. 2, an imaging direction of optical imaging by the first optical camera 26 and an imaging direction of radiography using the radiation emitting unit 12 and the radiation detector 20 are substantially the same direction. Here, the "substantially the same direction" may include a deviation to the extent that the registration with the radiation image can be achieved by subjecting image correction (geometric transformation) such as an affine transformation and a projective transformation to the optical image 30.

The position of the first optical camera 26 is not particularly limited, for example, as shown in Fig. 2, the first optical camera 26 may be attached to substantially the same surface as an irradiation opening of the radiation R of the radiation emitting unit 12, or may be attached to the wall surface of the imaging room, or the like. However, since it is preferable that the entire subject H can be optically imaged in order to specify the joint point, which will be described later, it is preferable that the first optical camera 26 is attached to substantially the same surface as the irradiation opening of the radiation R and below the irradiation opening of the radiation R. In addition, it is preferable that an optical axis Ao of the first optical camera 26 is substantially parallel to the irradiation axis Ar of the radiation R emitted from the radiation source 13.

In addition, it is assumed that a positional relationship between the radiation source 13 and the first optical camera 26 is predetermined. As shown in Fig. 2, for example, the positional relationship is represented by an interval dz in the Z direction and an interval dx (not shown) in the X direction between the irradiation axis Ar of the radiation R emitted from the radiation source 13 and the optical axis Ao of the first optical camera 26, and an interval dy in the Y direction between the radiation source 13 and the first optical camera 26. In addition, the intervals dx, dy, and dz representing the positional relationship and the angle of view ω of the first optical camera 26 are stored in the storage unit 52 in advance.

The second optical camera 28 generates a determination optical image 34 in which the subject H is optically imaged from a direction different from the imaging direction of the optical image 30 by the first optical camera 26 and whether the subject H takes predetermined positioning is determined (details will be described later). Fig. 2 shows an example in which the first optical camera 26 captures an image from the back side of the subject H, whereas the second optical camera 28 captures an image from the top side of the subject H.

The imaging apparatus 10 may comprise a control device that controls the overall operation of the imaging apparatus 10 in response to an instruction from the console 50 and the user (not shown). Specifically, the control device acquires image data indicating the radiation image generated by the radiation detector 20 and outputs the image data to the console 50. In addition, the control device acquires the optical image 30 of the subject H captured by the first optical camera 26 and the determination optical image 34 of the subject H captured by the second optical camera 28, and outputs the optical image 30 and the determination optical image 34 to the console 50.

The control device is composed of, for example, a central processing unit (CPU), a read only memory (ROM), a random access memory (RAM), a storage medium, an interface (I/F) unit, and an operation unit, which are not shown. The control device exchanges various types of information with the console 50 via the I/F unit.

By the way, in the radiography, it is desired to obtain a high-quality radiation image in which an imaging part of the subject H is appropriately imaged. For this purpose, it is important to perform registration of the radiation emitting unit 12 (radiation source 13), the radiation detector 20, and the subject H. In particular, in a case in which at least one position of the radiation emitting unit 12 or the radiation detector 20 is movable as described above, such registration is important. In addition, it is required to perform the positioning of the posture of the subject H as predetermined in the guideline or the like.

Therefore, the console 50 according to the present embodiment supports high-quality radiography by using the optical image 30 obtained by the first optical camera 26 and the determination optical image 34 obtained by the second optical camera 28.

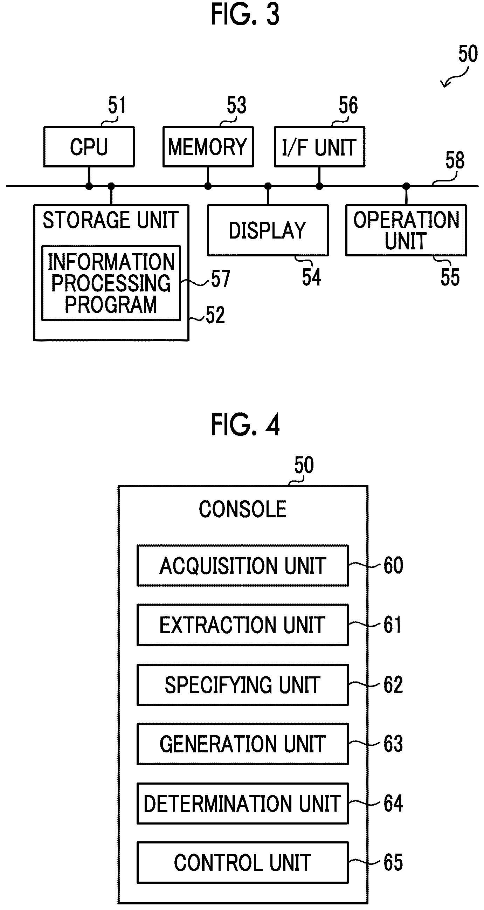

First, an example of a hardware configuration of the console 50 will be explained with reference to Fig. 3. As shown in Fig. 3, the console 50 includes a central processing unit (CPU) 51, a non-volatile storage unit 52, and a memory 53 as a temporary storage region. In addition, the console 50 includes a display 54 such as a liquid crystal display, an operation unit 55 such as a touch panel, a keyboard, and a mouse, and an interface (I/F) unit 56. The I/F unit 56 performs wired or wireless communication with the imaging apparatus 10, the RIS 6, and other external devices. The CPU 51, the storage unit 52, the memory 53, the display 54, the operation unit 55, and the I/F unit 56 are connected to each other via a bus 58 such as a system bus and a control bus, so that various types of information can be exchanged.

The storage unit 52 is realized by, for example, a storage medium such as a hard disk drive (HDD), a solid state drive (SSD), and a flash memory. In the storage unit 52, an information processing program 57 in the console 50 is stored. The CPU 51 reads out the information processing program 57 from the storage unit 52, develops the information processing program 57 into the memory 53, and executes the developed information processing program 57. The CPU 51 is an example of a processor of the present disclosure. As the console 50, for example, a personal computer, a server computer, a smartphone, a tablet terminal, or a wearable terminal can be appropriately applied.

Next, an example of a functional configuration of the console 50 will be explained with reference to Fig. 4. As shown in Fig. 4, the console 50 includes an acquisition unit 60, an extraction unit 61, a specifying unit 62, a generation unit 63, a determination unit 64, and a control unit 65. The CPU 51 executes the information processing program 57 to function as each functional unit of the acquisition unit 60, the extraction unit 61, the specifying unit 62, the generation unit 63, the determination unit 64, and the control unit 65.

### (Specification of target imaging region)

First, a method of specifying a target imaging region used for registration of the radiation emitting unit 12 (radiation source 13), the radiation detector 20, and the subject H will be described. The target imaging region is a region including a target part of radiography, and for example, in a case of imaging a chest, a region near the chest is the target imaging region, in a case of imaging a knee joint, a region near the knee is the target imaging region, and in a case of imaging a head, a region near the head is the target imaging region.

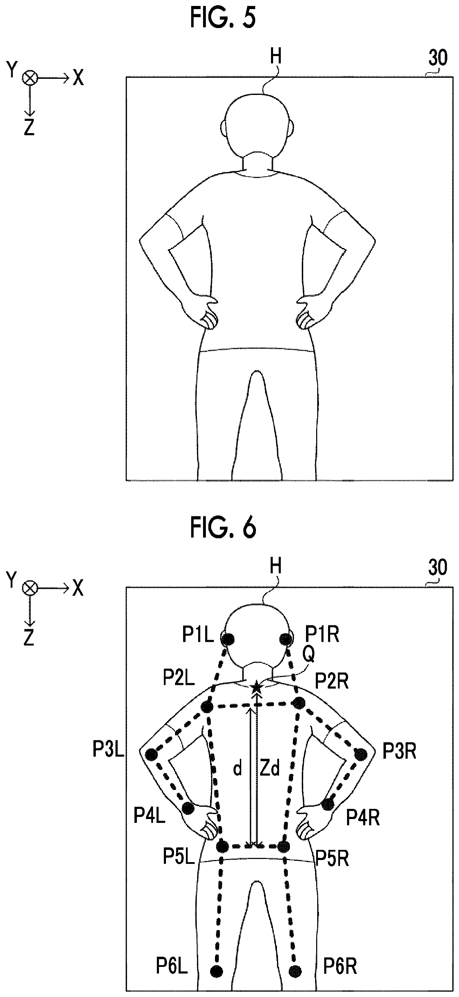

The acquisition unit 60 acquires at least one optical image 30 obtained by optical imaging of the subject H with the first optical camera 26. Fig. 5 shows an example of the optical image 30 captured by the first optical camera 26 in Fig. 2. In the optical image 30 of Fig. 5, the subject H is imaged from the back side.

The extraction unit 61 extracts the feature points of the subject H based on the optical image 30 acquired by the acquisition unit 60. In Fig. 6, a plurality of feature points P1L to P6L and P1R to P6R extracted from the optical image 30 of Fig. 5 are indicated by black dots. The feature points P1L to P6L and P1R to P6R correspond to joint points of the subject H, which are the ear, the shoulder, the elbow, the wrist, the waist, and the knee, respectively. Hereinafter, in a case in which the plurality of feature points P1L to P6L and P1R to P6R are not distinguished, the feature points are simply referred to as "feature point P", and the feature point corresponding to each joint point is referred to as "feature point of (joint point name)". As a method of extracting the feature point P (joint point), a known posture estimation technique or the like can be appropriately applied.

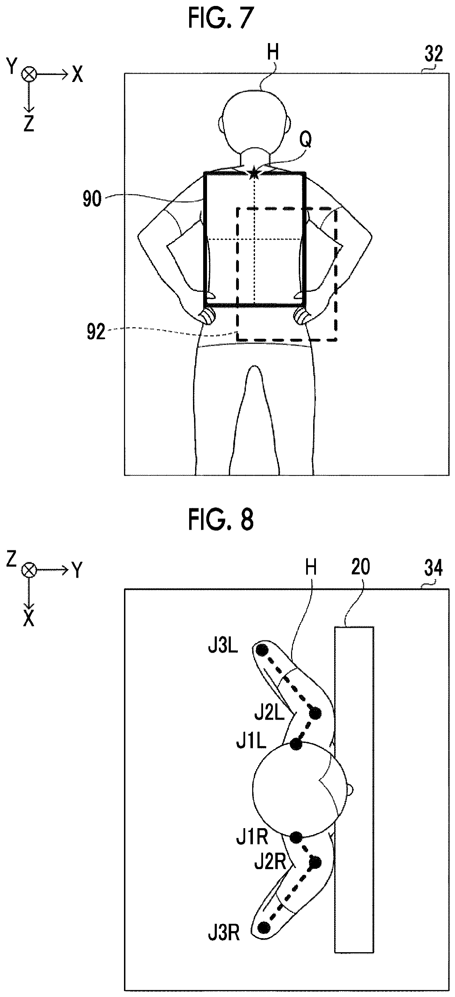

The specifying unit 62 specifies a target imaging region 90 that is a target in a case in which the subject H undergoes radiography from a direction substantially the same as the imaging direction of the optical imaging in the optical image 30, based on the feature point P extracted by the extraction unit 61. Fig. 7 shows a target imaging region 90 specified from the feature point P in the optical image 30 of Fig. 7 by a solid line rectangle.

Specifically, as shown in Fig. 6, the extraction unit 61 extracts a plurality of feature points P of the subject H based on the optical image 30, and the specifying unit 62 specifies the target imaging region 90 based on a relative positional relationship among the plurality of feature points P. For example, first, the specifying unit 62 specifies at least one predetermined reference feature point among the plurality of feature points P1L to P6L and P1R to P6R. In a case of imaging the chest as in the present embodiment, the specifying unit 62 specifies the feature points P2L and P2R of the shoulder and the feature points P5L and P5R of the waist as the reference feature points. Which of the feature points P correspond to the feature points P2L and P2R of the shoulder and to the feature points P5L and P5R of the waist can be determined based on the relative positional relationship among the plurality of feature points P.

It should be noted that which feature point P is set as the reference feature point may be determined in advance, may be optionally set by the user, or may be determined corresponding to the imaging part. For example, in the imaging of the head, the feature point of the eye, the feature point of the ear, and the like are appropriate as the reference feature point, and in the imaging of the knee joint, the feature point of the waist, the feature point of the knee, the feature point of the ankle, and the like are appropriate as the reference feature point. Therefore, the specifying unit 62 may acquire imaging part information indicating the imaging part of the radiography included in the imaging order acquired from the RIS 6 or the like, and specify the reference feature point corresponding to the acquired imaging part information. The type of the reference feature point corresponding to the imaging part information may be stored in the storage unit 52 in advance, for example.

Next, the specifying unit 62 specifies the target imaging region 90 based on the specified reference feature point. In the example of Fig. 6, the specifying unit 62 calculates a distance d in the cranio-caudal direction (Z direction) between the midpoint of the line segment (shown by a dotted line) connecting the feature points P2L and P2R of the shoulder and the midpoint of the line segment (shown by a dotted line) connecting the feature points P5L and P5R of the waist. In addition, the specifying unit 62 calculates a distance Zd obtained by multiplying the distance d by a predetermined coefficient, and specifies a point Q (shown by a star mark) separated from the midpoint of the line segment connecting the feature points P5L and P5R of the waist to the head direction by the distance Zd. The coefficient used for calculating the distance Zd may be optionally determined by the user anatomically and/or statistically, for example. In addition, for example, the machine learning model may be derived by unsupervised learning that is trained in advance using a combination of the optical image 30 for learning and the target imaging region 90 as learning data. In a case of imaging the chest, the point Q corresponds to the prominent vertebra.

Then, the specifying unit 62 specifies the specified point Q as the center of the upper side and a rectangular region having a size corresponding to the detection surface 20A of the radiation detector 20 as the target imaging region 90. The size of the target imaging region 90 (that is, the size corresponding to the detection surface 20A of the radiation detector 20) is obtained by geometric calculation using a source to image receptor distance (SID) that is a distance between the radiation source 13 and the detection surface 20A of the radiation detector 20 (see Fig. 2). As the value of the SID, for example, data stored in the storage unit 52 in advance or the like may be used on the premise that the imaging apparatus 10 is used in a state in which a predetermined appropriate SID is secured.

In addition, for example, the SID value may be determined by using an actually measured value measured by a distance measurement sensor such as a laser imaging detection and ranging or light detection and ranging (LIDAR), a time of flight (TOF) camera, and a stereo camera. The LIDAR and the TOF camera emit light, such as infrared light and visible light, and measure a distance based on a time until the reflected light is received or a phase change between the emitted light and the received light. The LIDAR measures a distance to an object to be measured by disposing a plurality of laser light emitters in a vertical direction and allowing each of the emitters to perform horizontally scanning (rotating). The TOF camera measures the distance to the object to be measured by emitting diffused light. The stereo camera measures the distance to the object to be measured by using a principle of triangulation based on a plurality of images obtained by imaging the object to be measured in different directions.

The generation unit 63 generates the superimposed image 32 in which the target imaging region 90 specified by the specifying unit 62 is superimposed on the optical image 30. In addition, as indicated by a broken line in Fig. 7, the generation unit 63 may further superimpose an irradiation field 92 of the radiation R emitted from the radiation emitting unit 12 on the superimposed image 32. The irradiation field 92 of the radiation R is obtained by, for example, a geometric calculation using the value of the SID, the positional relationship (intervals dx, dy, and dz) between the radiation source 13 and the first optical camera 26, the angle of view ω of the first optical camera 26, and the like stored in the storage unit 52 (see Fig. 2).

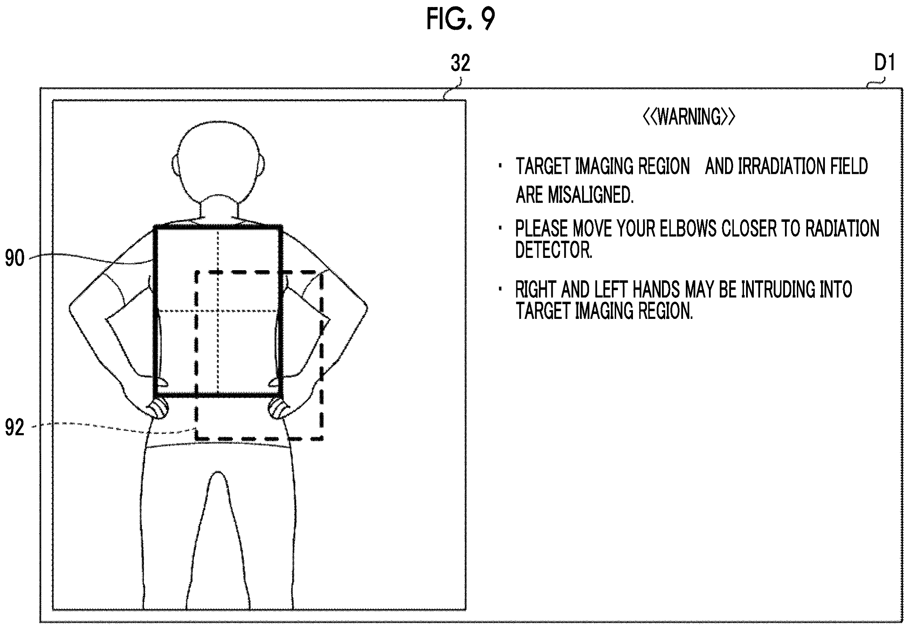

The control unit 65 performs control of displaying the superimposed image 32 generated by the generation unit 63 on the display 54. Fig. 9 shows an example of a screen D displayed on the display 54 by the control unit 65. The screen D1 includes the superimposed image 32 in which the target imaging region 90 and the irradiation field 92 of the radiation R are superimposed on the optical image 30. In the example of Fig. 9, the target imaging region 90 and the irradiation field 92 are misaligned, and in a case in which the radiography is performed in this state, it is not possible to obtain an appropriate radiation image. The user checks the screen D1 and moves at least one of the radiation emitting unit 12, the radiation detector 20, or the subject H such that the target imaging region 90 and the irradiation field 92 overlap each other, thereby performing the registration.

In addition, the control unit 65 may perform control to issue a warning in a case in which the coordinates of the target imaging region 90 and the coordinates of the irradiation field 92 are compared with each other in the superimposed image 32 and the difference between the coordinates is equal to or greater than a predetermined threshold value (that is, in a case in which the target imaging region 90 and the irradiation field 92 are largely misaligned). The screen D1 of Fig. 9 includes a warning message indicating that the target imaging region 90 and the irradiation field 92 are misaligned.

In a case in which the feature point P cannot be extracted and the target imaging region 90 cannot be specified at this point in time, it is considered that the positions of the radiation emitting unit 12, the radiation detector 20, and the subject H, the posture and positioning of the subject H, and the like are not appropriate. Therefore, the control unit 65 may perform control of displaying a notification on the display 54, for example, to prompt appropriate registration and positioning.

### (Appropriateness determination of positioning)

Next, a method of determining whether the posture of the subject H has been positioned as predetermined in a guideline or the like will be described.

The determination unit 64 determines whether the subject H is in the predetermined position based on the positional relationship among the plurality of feature points P extracted by the extraction unit 61. For example, in the example of Fig. 6, in a case in which the length of the line segment connecting the feature points P2L and P2R of the shoulder is less than a predetermined threshold value, there is a possibility that the subject H is not facing the radiation detector 20 and is facing an inclined direction. In addition, for example, in a case in which a line segment connecting the feature points P2L and P2R of the shoulder is inclined at an angle equal to or greater than a predetermined threshold value, there is a possibility that the subject H is not facing the radiation detector 20 and is facing the inclined direction. Therefore, the determination unit 64 determines whether the subject H is in the predetermined position by determining whether the plurality of feature points P extracted by the extraction unit 61 maintain the predetermined positional relationship.

In addition, the appropriateness determination of the positioning may be performed based on at least one determination optical image 34 obtained by the second optical camera 28 by optical imaging of the subject H from a direction different from the imaging direction of the optical image 30. In this case, the acquisition unit 60 acquires the determination optical image 34 obtained by the second optical camera 28. Fig. 8 shows an example of the determination optical image 34 captured by the second optical camera 28 of Fig. 2. In the determination optical image 34 of Fig. 8, the subject H is imaged from the top side of the head.

The extraction unit 61 extracts a plurality of determination feature points of the subject H based on the determination optical image 34 acquired by the acquisition unit 60. In Fig. 8, a plurality of determination feature points J1L to J3L and J1R to J3R extracted from the determination optical image 34 are indicated by black dots. The determination feature points J1L to J3L and J1R to J3R correspond to the joint points of the subject H of the ear, shoulder, and elbow, respectively. Hereinafter, in a case in which the plurality of determination feature points J1L to J3L and J1R to J3R are not distinguished, the determination feature points are simply referred to as "determination feature point J", and the determination feature point corresponding to each joint point is referred to as "determination feature point of (joint point name)". As a method of extracting the determination feature point J (joint point), a known posture estimation technique or the like can be appropriately applied.

The determination unit 64 determines whether the subject H takes predetermined positioning based on the positional relationship among the plurality of determination feature points J extracted by the extraction unit 61. For example, in the imaging of the chest, a posture in which the shoulder and the elbow are brought as close as possible to the detection surface 20A of the radiation detector 20 is determined in guidelines or the like. Therefore, in a case in which the line segment connecting the shoulder determination feature point J2L (J2R) and the elbow determination feature point J3L (J3R) is inclined at an angle equal to or greater than a predetermined threshold value, there is a possibility that the subject H has not sufficiently brought the elbow close to the detection surface 20A. Therefore, the determination unit 64 determines whether the subject H is in the predetermined position by determining whether the plurality of determination feature points J extracted by the extraction unit 61 maintain the predetermined positional relationship.

The control unit 65 performs control of issuing a warning in a case in which the determination unit 64 determines that the subject H does not take predetermined positioning based on at least one of the feature point P or the determination feature point J. The screen D1 of Fig. 9 includes a warning message that warns that the subject H may not sufficiently bring the elbow close to the detection surface 20A and that the elbow is brought close to the radiation detector 20.

In addition, the determination unit 64 may determine whether a feature point other than a predetermined feature point among the plurality of feature points P is included in the target imaging region 90 or is located (that is, is close) within a predetermined range from the target imaging region 90. For example, in a case in which an unnecessary portion overlaps the desired part for the radiography, there is a possibility that an appropriate radiation image cannot be obtained.

For example, in the example of Fig. 7, the fingertip of the subject H is excessively included in the target imaging region 90 that is an undesirable state. In a case of imaging the chest, the determination unit 64 determines whether the feature points other than the feature points P2L and P2R of the shoulder and the feature points P5L and P5R of the waist are included in or close to the target imaging region 90. As a result, it is determined that the feature points P4L and P4R of the wrist are located (that is, are close to each other) within a predetermined range from the target imaging region 90.

in a case in which the determination unit determines that a feature point other than the predetermined feature point among the plurality of feature points P is included in the target imaging region 90 or is located within the predetermined range from the target imaging region 90, the control unit 65 performs control to issue a warning. The screen D1 of Fig. 9 includes a warning message indicating that the right hand (the feature point P4R of the wrist) and the left hand (the feature point P4L of the wrist) may be intruding into the target imaging region 90.

The determination unit 64 may determine whether an unnecessary portion is included in the target imaging region 90 by a known image recognition technique, instead of determining whether the feature point P is included in the target imaging region 90 or is located within a predetermined range from the target imaging region 90. For example, in a case in which only the color of the examination suit is included in the target imaging region 90 in the optical image 30 and the color of the skin is detected, it may be determined that an unnecessary portion is included in the target imaging region 90.

The specification processing and the appropriateness determination processing of the target imaging region are repeatedly performed each time the optical image 30 is updated.

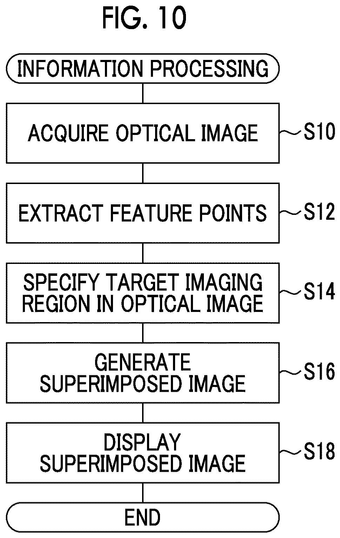

Next, an action of the console 50 according to the present embodiment will be described with reference to Fig. 10. In the console 50, the CPU 51 executes the information processing program 57 to execute information processing shown in Fig. 10. The information processing is executed, for example, in a case in which the user gives an instruction to start execution via the operation unit 55.

In step S10, the acquisition unit 60 acquires at least one optical image 30 obtained by optical imaging of the subject H with the first optical camera 26. In step S12, the extraction unit 61 extracts the feature points P of the subject H based on the optical image 30 acquired in step S10. In step S14, the specifying unit 62 specifies the target imaging region 90 that is a target in a case in which the subject H undergoes radiography from a direction substantially the same as the imaging direction of the optical imaging in the optical image 30, based on the feature point P extracted in step S12.

In step S16, the generation unit 63 generates the superimposed image 32 in which the target imaging region 90 specified in step S14 is superimposed on the optical image 30 acquired in step S10. In step S18, the control unit 65 performs control of displaying the superimposed image 32 generated in step S16 on the display 54, and ends the present information processing.

As described above, the console 50 according to one aspect of the present disclosure comprises at least one processor, and the processor acquires at least one optical image 30 obtained by optical imaging of the subject H, extracts the feature point P of the subject H based on the optical image 30, specifies a target imaging region 90 that is a target in a case in which the subject H undergoes radiography from a direction substantially the same as an imaging direction of the optical imaging in the optical image 30 based on the feature point P, generates a superimposed image 32 in which the target imaging region 90 is superimposed on the optical image 30, and performs control of displaying the superimposed image 32 on the display 54.

That is, with the console 50 according to the present embodiment, it is possible to specify the radiation emitting unit 12 (radiation source 13), the radiation detector 20, and the target imaging region 90 used for the registration of the subject H. Therefore, the radiation emitting unit 12 (radiation source 13), the radiation detector 20, and the subject H can be appropriately registered, and high-quality radiography can be supported.

In the above-described embodiment, although the optical image 30 obtained by the first optical camera 26 and the determination optical image 34 obtained by the second optical camera 28 are visible light images, the present disclosure is not limited thereto. For example, at least one of the optical image 30 obtained by the first optical camera 26 or the determination optical image 34 obtained by the second optical camera 28 may be a distance image representing a distance to the subject H. In this case, as the first optical camera 26 and the second optical camera 28, laser imaging detection and ranging or light detection and ranging (LIDAR), a time of flight (TOF) camera, a stereo camera, and the like can be appropriately applied.

The feature point P and the determination feature point J can be extracted by the distance image. Therefore, by using the above-described feature points, it is also possible to perform the appropriateness determination of specifying and positioning the target imaging region 90.

In addition, a combination in which the first optical camera 26 is a digital camera that obtains a visible light image and the second optical camera 28 is a three-dimensional camera that obtains a distance image can also be used. In addition, as the first optical camera 26, both a digital camera that obtains a visible light image and a three-dimensional camera that obtains a distance image may be applied, and both the visible light image and the distance image may be used for the appropriateness determination of specifying and positioning the target imaging region.

In addition, in the above-described embodiment, although the specifying unit 62 specifies the target imaging region 90 by using a formula including the relative positional relationship among the feature points P and a predetermined coefficient, and the generation unit 63 generates the superimposed image 32, the present disclosure is not limited thereto. For example, the specifying unit 62 may specify the target imaging region 90 by using a trained model that is trained in advance to receive at least one of the optical image 30 or the feature point P as an input and to output the target imaging region 90. In this case, the generation unit 63 may generate the superimposed image 32 by superimposing the target imaging region 90 specified using the trained model on the optical image 30. In this case, the optical image to be input may be a visible light image, a distance image, or both.

In addition, for example, the specifying unit 62 may integrally perform the specification of the target imaging region 90 and the generation of the superimposed image 32. For example, the generation unit 63 may generate the superimposed image 32 by using a trained model that is trained in advance to receive at least one of the optical image 30 or the feature point P as an input and to output the superimposed image 32. In this case, the optical image to be input may be a visible light image, a distance image, or both.

In addition, in each embodiment, for example, as hardware structures of processing units that execute various types of processing, such as the acquisition unit 60, the extraction unit 61, the specifying unit 62, the generation unit 63, the determination unit 64, and the control unit 65, various processors shown below can be used. As described above, in addition to the CPU that is a general-purpose processor that executes software (program) to function as various processing units, the various processors include a programmable logic device (PLD) that is a processor whose circuit configuration can be changed after manufacture, such as a field programmable gate array (FPGA), and a dedicated electric circuit that is a processor whose circuit configuration is designed for exclusive use in order to execute specific processing, such as an application-specific integrated circuit (ASIC).

One processing unit may be composed of one of the various processors or may be composed of a combination of two or more processors of the same type or different types (for example, a combination of a plurality of FPGAs or a combination of a CPU and an FPGA). In addition, a plurality of processing units may be composed of one processor.

A first example of the configuration in which the plurality of processing units are configured by one processor is a form in which one processor is configured by a combination of one or more CPUs and the software and this processor functions as the plurality of processing units, as represented by computers, such as a client and a server. Second, as represented by a system-on-chip (SoC) or the like, there is a form in which the processor is used in which the functions of the entire system which includes the plurality of processing units are implemented by a single integrated circuit (IC) chip. In this way, as the hardware structure, the various processing units are configured by one or more of the various processors described above.

Further, as the hardware structure of these various processors, more specifically, it is possible to use an electric circuit (circuitry) in which circuit elements such as semiconductor elements are combined.

In addition, in the above-described embodiment, an aspect in which the information processing program 57 in the console 50 is stored in the storage unit 52 in advance has been explained, the present disclosure is not limited thereto. The information processing program 57 may be provided in a form in which the information processing program 57 is recorded in recording mediums, such as a compact disc read only memory (CD-ROM), a digital versatile disc read only memory (DVD-ROM), and a universal serial bus (USB) memory. In addition, a form may be adopted in which the information processing program 57 is downloaded from an external devices via the network. Furthermore, the technology of the present disclosure includes, in addition to the program, a storage medium that stores the program in a non-transitory manner.

In the technology of the present disclosure, the embodiment and the examples described above can be combined as appropriate. The above-described contents and the above-shown contents are detailed descriptions for parts according to the technology of the present disclosure, and are merely examples of the technology of the present disclosure. For example, the above description related to the configuration, the function, the operation, and the effect is the description related to the examples of the configuration, the function, the operation, and the effect of the parts according to the technology of the present disclosure. As a result, it goes without saying that unnecessary parts may be deleted, new elements may be added, or replacements may be made with respect to the above-described contents and the above-shown contents within a range that does not deviate from the gist of the technology of the present disclosure.

In regard with the above-described embodiment, the following supplementary notes are further disclosed.

### [Supplementary Note 1]

An information processing apparatus comprising at least one processor,
in which the processor is configured to:
acquire at least one optical image obtained by optical imaging of a subject;
extract a feature point of the subject based on the optical image;
specify a target imaging region that is a target in a case in which the subject undergoes radiography from a direction substantially the same as an imaging direction of the optical imaging in the optical image, based on the feature point;
generate a superimposed image in which the target imaging region is superimposed on the optical image; and
perform control of displaying the superimposed image on a display.

### [Supplementary Note 2]

The information processing apparatus according to Supplementary Note 1,
in which the processor is configured to:
extract a plurality of feature points of the subject based on the optical image; and
specify the target imaging region based on a relative positional relationship among the plurality of feature points.

### [Supplementary Note 3]

The information processing apparatus according to Supplementary Note 2,
in which the processor is configured to:
specify at least one predetermined reference feature point among the plurality of feature points; and
specify the target imaging region based on the reference feature point.

### [Supplementary Note 4]

The information processing apparatus according to Supplementary Note 2 or 3,
in which the processor is configured to:
acquire imaging part information indicating an imaging part of the radiography; and
specify the reference feature point corresponding to the imaging part information.

### [Supplementary Note 5]

The information processing apparatus according to any one of Supplementary Notes 2 to 4,
in which the processor is configured to issue a warning in a case in which a feature point other than a predetermined feature point among the plurality of feature points is included in the target imaging region or is located within a predetermined range from the target imaging region.

### [Supplementary Note 6]

The information processing apparatus according to any one of Supplementary Notes 2 to 5,
in which the processor is configured to:
determine whether the subject takes predetermined positioning based on a positional relationship among the plurality of feature points; and
issue a warning in a case in which it is determined that the subject does not take predetermined positioning.

### [Supplementary Note 7]

The information processing apparatus according to any one of Supplementary Notes 1 to 6,
in which the processor is configured to generate the superimposed image using a trained model that is trained in advance to receive at least one of the optical image or the feature point as an input and to output the target imaging region or the superimposed image.

### [Supplementary Note 8]

The information processing apparatus according to any one of Supplementary Notes 1 to 7,
in which the processor is configured to:
acquire at least one determination optical image obtained by optical imaging of the subject from a direction different from the imaging direction of the optical image;
extract a plurality of determination feature points of the subject based on the determination optical image;
determine whether the subject takes predetermined positioning based on a positional relationship among the plurality of determination feature points; and
issue a warning in a case in which it is determined that the subject does not take predetermined positioning.

### [Supplementary Note 9]

The information processing apparatus according to any one of Supplementary Notes 1 to 8, in which the optical image is at least one of a visible light image or a distance image representing a distance to the subject.

### [Supplementary Note 10]

An information processing method including:
acquiring at least one optical image obtained by optical imaging of a subject;
extracting a feature point of the subject based on the optical image;
specifying a target imaging region that is a target in a case in which the subject undergoes radiography from a direction substantially the same as an imaging direction of the optical imaging in the optical image, based on the feature point;
generating a superimposed image in which the target imaging region is superimposed on the optical image; and
performing control of displaying the superimposed image on a display.

### [Supplementary Note 11]

An information processing program that causes a computer to execute a process including:
acquiring at least one optical image obtained by optical imaging of a subject;
extracting a feature point of the subject based on the optical image;
specifying a target imaging region that is a target in a case in which the subject undergoes radiography from a direction substantially the same as an imaging direction of the optical imaging in the optical image, based on the feature point;
generating a superimposed image in which the target imaging region is superimposed on the optical image; and
performing control of displaying the superimposed image on a display.

The disclosure of JP2023-025466 filed on February 21, 2023, is incorporated in the present specification by reference. All of the documents, the patent applications, and the technical standards described in the present specification are incorporated into the present specification by reference to the same extent as in a case in which each of the documents, the patent applications, and the technical standards are specifically and individually stated to be described by reference.

## Claims

1. An information processing apparatus comprising at least one processor, wherein the processor is configured to:
acquire at least one optical image obtained by optical imaging of a subject;
extract a feature point of the subject based on the optical image;
specify a target imaging region that is a target in a case in which the subject undergoes radiography from a direction substantially the same as an imaging direction of the optical imaging in the optical image, based on the feature point;
generate a superimposed image in which the target imaging region is superimposed on the optical image; and
perform control of displaying the superimposed image on a display.

2. The information processing apparatus according to claim 1, wherein the processor is configured to:
extract a plurality of feature points of the subject based on the optical image; and
specify the target imaging region based on a relative positional relationship among the plurality of feature points.

3. The information processing apparatus according to claim 2, wherein the processor is configured to:
specify at least one predetermined reference feature point among the plurality of feature points; and
specify the target imaging region based on the reference feature point.

4. The information processing apparatus according to claim 3, wherein the processor is configured to:
acquire imaging part information indicating an imaging part of the radiography; and
specify the reference feature point corresponding to the imaging part information.

5. The information processing apparatus according to claim 1, wherein the processor is configured to generate the superimposed image using a trained model that is trained in advance to receive at least one of the optical image or the feature point as an input and to output the target imaging region or the superimposed image.

6. The information processing apparatus according to claim 2, wherein the processor is configured to issue a warning in a case in which a feature point other than a predetermined feature point among the plurality of feature points is included in the target imaging region or is located within a predetermined range from the target imaging region.

7. The information processing apparatus according to claim 2, wherein the processor is configured to:
determine whether the subject takes predetermined positioning based on a positional relationship among the plurality of feature points; and
issue a warning in a case in which it is determined that the subject does not take predetermined positioning.

8. The information processing apparatus according to claim 1, wherein the processor is configured to:
acquire at least one determination optical image obtained by optical imaging of the subject from a direction different from the imaging direction of the optical image;
extract a plurality of determination feature points of the subject based on the determination optical image;
determine whether the subject takes predetermined positioning based on a positional relationship among the plurality of determination feature points; and
issue a warning in a case in which it is determined that the subject does not take predetermined positioning.

9. The information processing apparatus according to claim 1, wherein the optical image is at least one of a visible light image or a distance image representing a distance to the subject.

10. An information processing method comprising:
acquiring at least one optical image obtained by optical imaging of a subject;
extracting a feature point of the subject based on the optical image;
specifying a target imaging region that is a target in a case in which the subject undergoes radiography from a direction substantially the same as an imaging direction of the optical imaging in the optical image, based on the feature point;
generating a superimposed image in which the target imaging region is superimposed on the optical image; and
performing control of displaying the superimposed image on a display.

11. An information processing program that causes a computer to execute a process comprising:
acquiring at least one optical image obtained by optical imaging of a subject;
extracting a feature point of the subject based on the optical image;
specifying a target imaging region that is a target in a case in which the subject undergoes radiography from a direction substantially the same as an imaging direction of the optical imaging in the optical image, based on the feature point;
generating a superimposed image in which the target imaging region is superimposed on the optical image; and
performing control of displaying the superimposed image on a display.
